# DEMANDE DE BREVET EUROPEEN

(11) **EP 0 689 848 A1**
(43) Date de publication de la demande: **03.01.1996**
(21) Numéro de dépôt: 95401494.0
(22) Date de dépôt: 23.06.1995
(51) Int. Cl.: A61M 15/00, A61B 5/00, G01N 15/14, G01F 1/66

(54) **Inhalateur de poudre**

(30) Priorité: 27.06.1994 FR 9407873
(71) Demandeur: COMMISSARIAT A L'ENERGIE ATOMIQUE, F-75015 Paris Cédex 15 (FR)
(72) Inventeur: Gaucher, Jean-Claude, F-91530 Le Val Saint Germain (FR); Ngo, Christian, F-78470 Saint Remy Les Chevreuses (FR); Roche, Pierre, F-94700 Maisons Alfort (FR); Thevenin, Jean-Claude, F-78140 Velizy-Villacoublay (FR)
(74) Mandataire: Dubois-Chabert, Guy

(57) **Abrégé**

L'invention concerne un dispositif pulvérisateur d'un médicament se présentant sous forme de poudre, comprenant un réservoir de gaz propulseur (3) et un réservoir pour le médicament (2) équipés de valves (8, 12) pour délivrer leur contenu en un jet vers une buse d'injection (7) sur laquelle s'adapte un embout buccal ou nasal (5), le dispositif comprenant en outre un organe de contrôle comprenant des moyens de dosage agissant sur les valves (8, 12) pour délivrer une quantité déterminée de médicament lors d'une pulvérisation et des moyens de blocage empêchant toute pulvérisation pendant une durée déterminée après une utilisation du dispositif. La poudre est constituée de microsphères et les moyens de dosage comportent des moyens de comptage des microsphères.

L'invention concerne également un ensemble doseur intelligent comprenant ce dispositif pulvérisateur de médicament et un capteur portable par un malade et mesurant le taux de l'un de ses constituant sanguins.

## Description

La présente invention concerne un dispositif d'administration de médicaments en poudre, par voie aérienne, dans des zones très vascularisées telles les poumons ou les cavités nasales. Ce pulvérisateur microdoseur automatique et programmable peut délivrer un aérosol constitué de microsphères dont la dimension est de l'ordre du micromètre. Il peut éventuellement être piloté par un capteur de mesure non invasive des constituants sanguins. L'ensemble miniaturisé forme alors un microsystème. L'invention s'applique particulièrement au suivi des malades insulinodépendants par la mesure du glucose et l'administration d'insuline.

L'administration d'insuline est encore, pour la majorité des patients souffrant d'un diabète insulinodépendant, très traumatisante. Celle-ci se fait par injection avec une seringue ou un stylo injecteur. Cette administration a lieu plusieurs fois par jour. Depuis les années 1970, des recherches ont porté sur le développement de pompes à insuline. Un certain nombre de modèles sont industrialisés et implantés dans la cavité péritonéale des patients. Leur durée de vie est comprise entre 3 et 5 ans. Ce dispositif contient un réservoir d'insuline de quelques cm³.

Les pompes à insuline posent actuellement deux problèmes importants. D'une part, à une température de 37°C, l'insuline en solution a tendance à précipiter lorsque la solution est agitée. La pompe étant implantée sur un malade, les conditions sont très favorables à la précipitation. D'autre part, le cathéter, qui permet de faire passer la solution du dispositif implanté dans le malade, est souvent très vite colonisé par les tissus humains. Ce problème de biocompatibilité conduit à un taux de bouchage de l'ordre de 10 à 15%.

Les problèmes évoqués ci-dessus peuvent être évités si l'insuline se présente sous forme solide et si son administration se fait par voie externe.

Lorsque le patient est en état d'hyperglycémie, l'insuline doit arriver dans le sang en moins d'une dizaine de minutes. Ce temps, relativement bref, implique que l'administration ne peut se faire que dans des zones fortement vascularisées. Celles-ci peuvent être la cavité péritonéale comme dans le cas des pompes à insuline mais il n'est pas possible d'accéder à cet endroit par voie externe. Les autres possibilités sont la cavité intranasale et les poumons.

Pour remédier aux inconvénients de l'art antérieur (administration d'insuline par injection ou par pompe), on propose selon la présente invention un dispositif permettant l'administration sous forme de poudre par l'intermédiaire de la cavité intranasale ou des poumons. La cavité intranasale est facilement accessible mais présente des inconvénients qui sont sa fragilité et le fait que sa perméabilité varie d'un individu à l'autre et de facteurs qui dépendent du temps (rhume, par exemple). La voie intrapulmonaire peut s'avérer plus intéressante dans ce contexte.

Il faut cependant prendre en considération le fait que l'insuline doit être administrée au malade à des doses strictement définies par le médecin traitant et à des intervalles également bien définis.

Le dispositif d'administration proposé est particulièrement bien adapté pour l'insuline mais il a une portée plus générale. Il peut être utile dans tous les cas où l'on souhaite administrer un médicament, sous forme de poudre ou d'aérosol, dans les poumons ou dans la cavité nasale de manière contrôlée. Même si la quantité de médicaments concernés n'est pas critique, le dispositif permet de faire une économie substantielle de produit en délivrant juste la dose nécessaire et en utilisant complètement les ressources du réservoir contenant le médicament. Il peut donc être utilisé pour administrer :
- des bronchodilatateurs prescrits contre l'asthme,
- des calcitonines pour l'ostéoporose,
- l'héparine pour le traitement de la maladie thromboembolique,
- les gonadotrophines,
- certaines protéines (traitement de la mucoviscidose, par exemple).

L'invention apporte une solution à ce problème en proposant un dispositif pulvérisateur d'un médicament se présentant sous forme de poudre, comprenant un réservoir de gaz propulseur et un réservoir pour le médicament équipés de valves pour délivrer leur contenu en un jet vers une buse d'injection sur laquelle s'adapte un embout buccal ou nasal, le dispositif comprenant en outre un organe de contrôle comprenant des moyens de dosage agissant sur les valves pour délivrer une quantité déterminée de médicament lors d'une pulvérisation et des moyens de blocage empêchant toute pulvérisation pendant une durée déterminée après une utilisation du dispositif, caractérisé en ce que la poudre étant constituée de microsphères, les moyens de dosage comportent des moyens de comptage des microsphères.

Le dispositif selon l'invention permet donc de doser très exactement la quantité d'insuline administrée.

Il peut comprendre également des moyens permettant de débloquer manuellement les moyens de blocage lorsque ceux-ci tombent en panne.

Avantageusement, les moyens de blocage sont assurés par un microprocesseur relié à des moyens de mémoire contenant des données relatives à la quantité de médicament à absorber et à sa fréquence d'administration, le microprocesseur gérant la commande des valves en fonction desdites données.

Dans ce cas, les moyens de mémoire peuvent également servir à stocker des informations relatives aux administrations de médicaments effectuées (quantité et fréquence).

Les moyens de dosage peuvent comprendre des moyens de focalisation aérodynamique des microsphères contenues dans ledit jet et des moyens de mesure optique de la quantité de microsphères qui y est contenue et permettant leur comptage.

Dans le cas d'un traitement à l'insuline de l'hyperglycémie, il est nécessaire de contrôler la quantité de glucose dans le sang du malade. La mesure ambulatoire du glucose est encore à ce jour traumatisante car le patient doit prélever une goutte de son sang. Il existe néanmoins des appareils de mesure non invasive du glucose mais ceux-ci ont une taille et un prix tels qu'ils sont réservés à l'usage en laboratoire. On peut citer pour exemple l'appareil commercialisé par la société FUTREX qui réalise une mesure en bout de doigt. Leur principe est basé sur une méthode utilisée depuis fort longtemps : la spectrophotométrie infrarouge. Le choix de l'infrarouge est dicté par le fait que le faisceau analyseur doit pouvoir traverser les tissus et que les molécules de glucose doivent posséder des raies d'absorption caractéristiques dans le domaine spectral utilisé.

Il entre dans le cadre de la présente invention d'associer le nouveau dispositif pulvérisateur décrit ci-dessus à un capteur, de préférence non invasif, destiné à mesurer le taux de glucose dans le sang. Cet ensemble forme ainsi un doseur intelligent qui gère la délivrance du médicament en fonction des résultats de l'analyse. Le capteur de glycémie sera de préférence contrôlé par un microprocesseur. Une base de données contiendra les informations nécessaires à la gestion du capteur, en particulier les codes de calcul du taux de glucose sanguin ainsi que les protocoles de communications. Le capteur disposera d'une alarme qui sera activée lorsque des seuils préétablis par le médecin traitant seront franchis. Les résultats mesurés seront transmis par voie hertzienne au microdoseur.

Préférentiellement la mesure du glucose sera effectuée par une méthode optique. Ainsi, il est envisageable de réaliser cette mesure par un spectrophotomètre dont les dimensions sont telles qu'il ne dépasse pas la taille d'une montre ou celle d'un objet dont le patient peut facilement se munir. La miniaturisation du dispositif, et par conséquent sa portabilité, associée au caractère non invasif de la mesure permettront un suivi permanent du malade, donc une diminution du temps de réaction en cas de crise.

Comme précédemment, cet ensemble est particulièrement bien adapté à l'administration d'insuline, mais il pourrait également être utilisé pour administrer un autre médicament en relation avec le taux de l'un des constituants du sang.

L'invention a donc aussi pour objet un ensemble doseur intelligent comprenant le nouveau dispositif pulvérisateur d'un médicament décrit ci-dessus, un capteur portable par un malade et permettant de mesurer, grâce à des moyens de mesure, le taux d'un constituant sanguin du malade, ce taux devant être contrôlé grâce audit médicament, des moyens de communication et d'alarme répartis sur le dispositif pulvérisateur et le capteur et permettant d'avertir le malade qu'il doit s'administrer son médicament lorsque le taux mesuré par le capteur devient critique.

L'invention sera mieux comprise et d'autres avantages et particularités apparaîtront à la lecture de la description qui va suivre, donnée à titre d'exemple non limitatif, accompagnée des dessins parmi lesquels :
- la figure 1 représente, de manière schématique et partiellement en coupe, un dispositif pulvérisateur de médicament selon l'invention,
- la figure 2 représente un compteur de particules utilisable pour le dispositif pulvérisateur selon l'invention,
- la figure 3 représente un ensemble doseur intelligent comprenant un pulvérisateur de médicament selon l'invention associée à un capteur porté par un malade,
- la figure 4 représente des schémas des circuits électroniques répartis sur le dispositif pulvérisateur et sur le capteur.

La description qui va suivre va porter sur un dispositif pulvérisateur d'insuline et sur un ensemble utilisant un tel dispositif pulvérisateur. Il s'agit d'une application particulièrement intéressante de l'invention. Cependant, comme il a été dit plus haut, l'invention ne se limite pas au cas de l'administration d'insuline et peut également s'appliquer à l'administration d'autres médicaments.

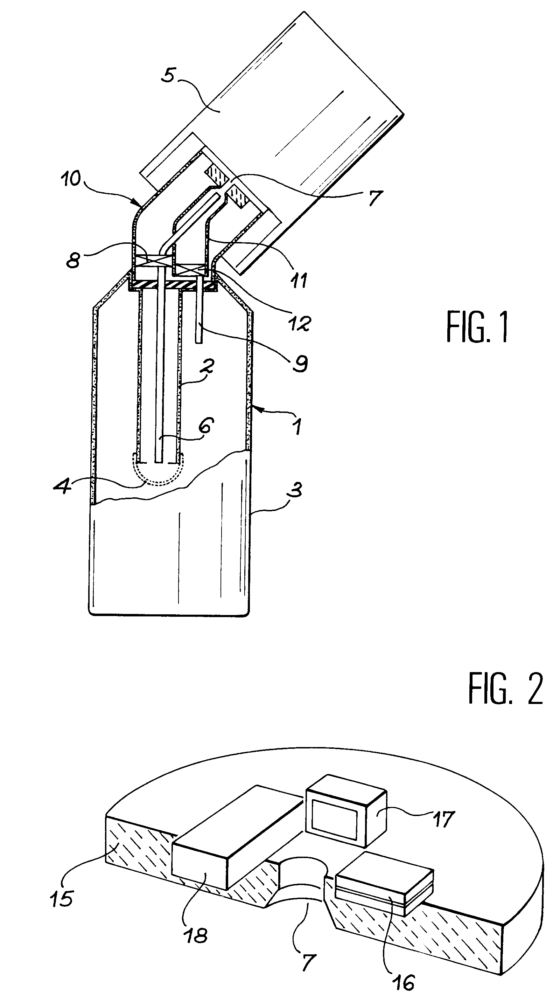

Le dispositif pulvérisateur représenté à la figure 1 comprend un ensemble 1, jetable ou reconditionnable, qui contient l'insuline sous forme de poudre à granulométrie connue et une réserve de gaz sous pression. Le réservoir à insuline 2 et le réservoir à gaz propulseur 3 forment un même ensemble. Le réservoir à insuline 2 est, par exemple, muni d'une embase frittée 4 de sorte que l'insuline se trouve à la pression du réservoir 3 contenant le gaz de chasse. Cette disposition est avantageuse car elle permet une agitation modérée de la poudre d'insuline après chaque prise, lors du rééquilibrage des pressions.

Un embout, qui peut être en plastique, permet d'administrer l'insuline soit dans les poumons, comme l'embout buccal 5 représenté, soit dans la cavité nasale. Cet élément est de préférence jetable pour des raisons d'hygiène.

Entre l'ensemble 1 et l'embout 5 on trouve une tête de contrôle 10 qui assure plusieurs fonctions. Elle contient une pile non représentée pour fournir l'énergie nécessaire à son fonctionnement.

La première fonction de la tête de contrôle est de doser très exactement la quantité d'insuline administrée. Pour réaliser ceci, la tête de contrôle comprend un système de focalisation aérodynamique des microsphères constituant la poudre, associé à un dispositif de mesure optique (technique inspirée de celle utilisée en cytométrie en flux) qui a été miniaturisé à l'extrême.

Un tube 6 plonge l'une de ses extrémités dans le fond du réservoir à insuline 2. Son autre extrémité aboutit en face de la buse d'injection de l'aérosol 7. Le tube 6 possède une valve 8 située dans la tête de contrôle 10.

Le gaz propulseur est amené jusqu'à la buse d'injection 7 par un tube composé d'une première partie 9 plongeant dans le réservoir 3 et d'une seconde partie 11 aboutissant à la buse d'injection 7. Les parties 9 et 11 sont raccordées entre elles par un ensemble 12 comprenant une valve et un détendeur. Comme le montre la figure 1, le tube 6, après la valve 8, pénètre dans la partie 11 du tube amenant le gaz propulseur pour se centrer sur l'axe de la buse d'injection 7. Cette disposition permet la focalisation aérodynamique des microsphères dans le microtrou d'injection constituant la buse d'injection.

L'ouverture de la valve 12 crée un courant gazeux qui s'échappe en flux laminaire par la buse d'injection 7. Ce flux entraîne et centre les particules d'insuline libérées par l'ouverture de la valve 8 dans le microtrou d'injection.

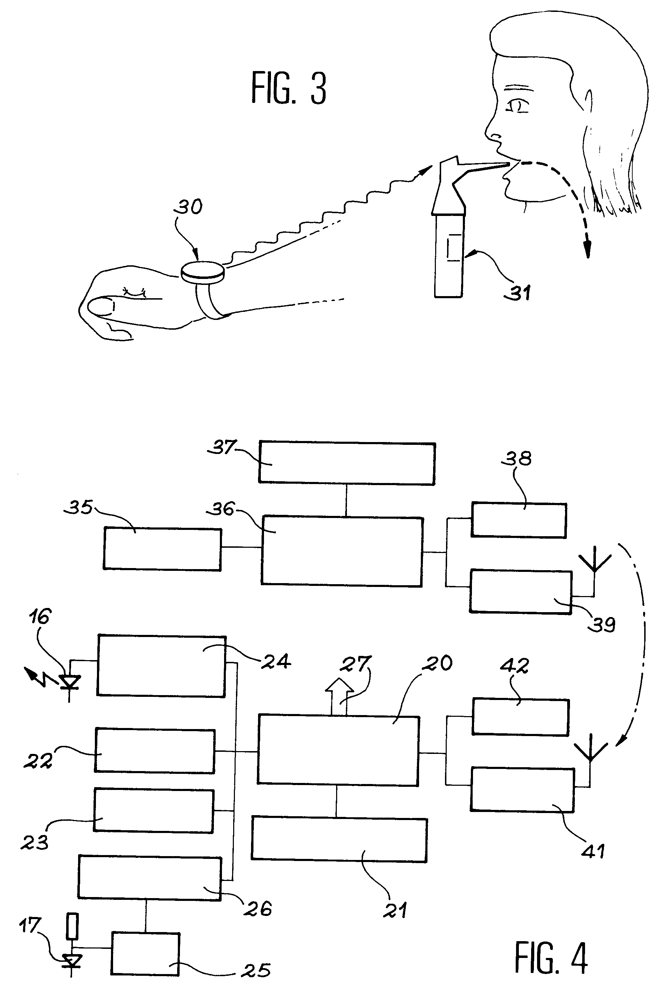

La figure 2 est une vue de détail montrant, en agrandissement la buse d'injection 7 du dispositif pulvérisateur selon l'invention. La buse 7 est un microtrou percé dans une plaque 15 fermant la tête de contrôle 10. A la sortie de la buse, le flux contenant les particules centrées intercepte le faisceau lumineux émis par une source collimatée 16. Dans un mode de réalisation préférentiel, cette source sera une diode laser. Un photodétecteur 17, placé par exemple à 90° de l'axe du faisceau lumineux, reçoit de la lumière diffusée par les particules émises par le dispositif pulvérisateur. Le photodétecteur délivre un signal représentatif du nombre de particules de médicament émises par le dispositif pulvérisateur. Il peut s'intégrer dans un circuit électronique du type représenté à la figure 4.

L'utilisation d'un absorbeur de faisceau lumineux 18 évite que la lumière non diffusée par les particules n'atteigne le photodétecteur 17 par des réflexions parasites, ce qui pourrait affecter la réponse du photodétecteur. L'absorbeur 18 est placé en vis-à-vis de la source 16, de l'autre côté du microtrou.

Lorsque le nombre de microsphères comptées est suffisant, la valve 8 est fermée. Au bout du temps considéré comme nécessaire à l'absorption par le patient des dernières particules d'insuline, la valve 12 est fermée à son tour. La valve 12 est équipée d'un détendeur qui assure une différence de pression positive entre le réservoir d'insuline et le flux de centrage. Cette légère surpression permet de contrôler le débit moyen des microsphères d'insuline.

Il peut être intéressant de contrôler le fonctionnement des valves par une mesure de la pression au niveau de l'embout buccal (ou nasal). Ce contrôle, destiné à assurer que l'injection a lieu pendant une phase d'aspiration du patient, garantit l'efficacité du traitement.

La seconde fonction de la tête de contrôle est liée à la sécurité et à l'enregistrement des données. Après l'administration du médicament, la tête de contrôle bloque le système pendant un temps prédéterminé par le médecin pour éviter une seconde administration accidentelle. Ce blocage peut néanmoins être enlevé manuellement par le patient dans le cas peu probable d'une panne.

L'enregistrement des données permet au médecin traitant, après lecture de la mémoire de la tête de contrôle rendue possible en se connectant sur le bus 27 d'accès au microprocesseur, de connaître la quantité, la date et l'heure d'administration du médicament. Cette fonction peut être très utile pour le suivi du malade ainsi que pour les statistiques médicales.

La tête de contrôle peut être achetée par le malade dans le cas d'un traitement de longue durée ou louée dans le cas de traitements ponctuels.

La figure 4 représente schématiquement l'électronique de la tête de contrôle. Celle-ci est gérée par un microprocesseur central 20 qui contrôle les différentes fonctions : celles liées à l'acquisition des informations, celles de commande des valves et de la source de lumière. Un circuit mémoire 21 contient une base de données relatives à l'administration d'insuline (quantité et fréquence d'administration) et permet de mémoriser les administrations réalisées. Le microprocesseur gère également un circuit 22 de commande de la valve 8 et un circuit 23 de commande de la valve 12. Il pilote également, grâce au circuit de pilotage 24, la diode laser 16 pour la faire fonctionner lors de l'administration de médicament. Il recueille les signaux de comptage transmis par le photodétecteur via l'amplificateur 25 et le circuit 26 de comptage des particules.

Comme il a été dit plus haut, l'invention permet d'associer un capteur pour la mesure en temps réel de la quantité de glucose dans le sang et le dispositif pulvérisateur programmable qui vient d'être décrit et qui contrôle parfaitement la quantité administrée, Le dispositif d'administration permet, à lui seul, de doser exactement la quantité de médicament administrée. Ceci permet donc une médication plus précise et une économie substantielle de produit. Le microdoseur pulvérisateur et le capteur de mesure des constituants sanguins peuvent être utilisés séparément.

La figure 3 illustre l'association d'un capteur 30 permettant la mesure du taux de glucose dans le sang d'un malade et d'un dispositif pulvérisateur 31 selon l'invention. Le capteur peut être porté par le malade à la manière d'une montre. La mesure du taux de glucose peut se faire de manière optique, en utilisant de préférence la spectroscopie infrarouge.

La partie supérieure de la figure 4 correspond aux circuits électroniques du capteur. Les données fournies par le dispositif optique de mesure du glucose 35 sont adressées au microprocesseur 36 qui gère ces données en fonction des informations contenues dans le circuit mémoire 37. En fonction du taux de glucose mesuré par le dispositif 35 et des informations contenues dans le circuit mémoire 37, le microprocesseur 36 déclenche, le cas échéant, un dispositif d'alarme. Pour cela, un signal d'alarme généré par le circuit d'alarme 38 est émis par l'émetteur 39 vers la tête de contrôle du dispositif pulvérisateur. Ce signal, se propageant par exemple sous forme d'ondes hertziennes, est reçu par un récepteur 41 du dispositif pulvérisateur. Il est transmis au circuit 42 qui déclenche une alarme pour avertir le malade qu'il doit s'administrer de l'insuline. Le signal est également pris en compte par le microprocesseur 20 et enregistré dans le circuit mémoire 21.

En fonction du taux de glucose mesuré et des informations contenues dans le circuit mémoire 37, le microprocesseur 36 peut calculer la dose d'insuline à administrer au malade et transmettre cette donnée au microprocesseur 20 du pulvérisateur. Le microprocesseur 20 peut alors commander les valves 22 et 23 de manière à prévoir la dose d'insuline exacte à administrer.

Le dispositif pulvérisateur peut en outre comporter un élément renseignant sur le niveau de remplissage du réservoir d'insuline.

## Revendications

1. Dispositif pulvérisateur d'un médicament se présentant sous forme de poudre, comprenant un réservoir de gaz propulseur (3) et un réservoir pour le médicament (2) équipés de valves (8, 12) pour délivrer leur contenu en un jet vers une buse d'injection (7) sur laquelle s'adapte un embout buccal ou nasal (5), le dispositif comprenant en outre un organe de contrôle comprenant des moyens de dosage agissant sur les valves (8, 12) pour délivrer une quantité déterminée de médicament lors d'une pulvérisation et des moyens de blocage empêchant toute pulvérisation pendant une durée déterminée après une utilisation du dispositif, caractérisé en ce que la poudre étant constituée de microsphères, les moyens de dosage comportent des moyens de comptage des microsphères.

2. Dispositif selon la revendication 1, caractérisé en ce qu'il comprend également des moyens permettant de débloquer manuellement les moyens de blocage lorsque ceux-ci tombent en panne.

3. Dispositif selon l'une des revendications 1 ou 2, caractérisé en ce que les moyens de blocage sont assurés par un microprocesseur (20) relié à des moyens de mémoire (21) contenant des données relatives à la quantité de médicament à absorber et à sa fréquence d'administration, le microprocesseur (20) gérant la commande des valves (22, 23) en fonction desdites données.

4. Dispositif selon la revendication 3, caractérisé en ce que les moyens de mémoire (21) servent également à stocker des informations relatives aux administrations de médicaments effectuées (quantité et fréquence).

5. Dispositif selon l'une quelconque des revendications 1 à 4, caractérisé en ce que les moyens de dosage comprennent des moyens de focalisation aérodynamique des microsphères contenues dans ledit jet et des moyens de mesure optique de la quantité de microsphères qui y est contenue et permettant leur comptage.

6. Dispositif selon la revendication 5, caractérisé en ce que, le dispositif comprenant un premier tube (11) amenant le gaz propulseur vers la buse d'injection (7) et un deuxième tube (6) amenant la poudre vers la buse d'injection, l'extrémité du deuxième tube est disposée à l'intérieur et dans l'axe du premier tube pour constituer lesdits moyens de focalisation aérodynamique.

7. Dispositif selon l'une des revendications 5 ou 6, caractérisé en ce que les moyens de mesure comprennent une source collimatée (16) émettant un faisceau lumineux en direction des microsphères focalisées aérodynamiquement dans ledit jet et un photodétecteur (17) recueillant une fraction du faisceau lumineux diffusée par les microsphères.

8. Dispositif selon la revendication 7, caractérisé en ce que la source collimatée (16) est une diode laser.

9. Dispositif selon l'une des revendications 7 ou 8, caractérisé en ce qu'il comprend aussi un absorbeur de lumière (18) disposé en vis-à-vis de la source collimatée (16), ledit jet passant entre la source et l'absorbeur.

10. Dispositif selon l'une quelconque des revendications 7 à 9, caractérisé en ce que, dans le cas où les moyens de blocage sont assurés par un microprocesseur (20), l'organe de contrôle comprend un circuit de comptage (26) des microsphères relié au photodétecteur (17) et délivrant au microprocesseur (20) un signal représentatif de la quantité de médicament administrée à chaque utilisation.

11. Dispositif selon l'une quelconque des revendications 7 à 10, caractérisé en ce que, dans le cas où les moyens de blocage sont assurés par un microprocesseur (20), l'organe de contrôle comprend un circuit de commande (24) de la source collimatée (16) géré par le microprocesseur (20).

12. Dispositif selon l'une quelconque des revendications 1 à 11, caractérisé en ce que le médicament est de l'insuline.

13. Ensemble doseur intelligent comprenant un dispositif pulvérisateur (31) d'un médicament selon l'une quelconque des revendications 1 à 11, un capteur (30) portable par un malade et permettant de mesurer, grâce à des moyens de mesure, le taux d'un constituant sanguin du malade, ce taux devant être contrôlé grâce audit médicament, des moyens de communication (39, 41) et d'alarme (38, 42) répartis sur le dispositif pulvérisateur et le capteur et permettant d'avertir le malade qu'il doit s'administrer son médicament lorsque le taux mesuré par le capteur devient critique.

14. Ensemble selon la revendication 13, caractérisé en ce que le capteur possède des moyens optiques de mesure du taux dudit constituant sanguin.

15. Ensemble selon la revendication 14, caractérisé en ce que les moyens optiques du capteur sont des moyens de spectroscopie infrarouge.

16. Ensemble selon l'une quelconque des revendications 13 à 15, caractérisé en ce que le capteur comprend un microprocesseur (36) relié à des moyens de mémoire (37) contenant des données relatives audit constituant sanguin et recevant le signal délivré par les moyens de mesure (35), le capteur comprenant encore un circuit d'alarme (38) et un circuit émetteur (39) d'un signal d'alarme, ces circuits étant gérés par le microprocesseur du capteur, le dispositif pulvérisateur possédant un circuit récepteur (41) du signal d'alarme émis par le capteur et un dispositif d'alarme (42) avertissant le malade en réponse au signal d'alarme.

17. Ensemble selon la revendication 16, caractérisé en ce que la communication entre l'émetteur (39) du capteur et le récepteur (41) du dispositif pulvérisateur se fait par ondes hertziennes.

18. Ensemble selon l'une quelconque des revendications 13 à 17, caractérisé en ce que, le dispositif de pulvérisation étant équipé d'un microprocesseur (20), le capteur calcule et transmet au dispositif pulvérisateur une information sur la quantité de médicament à administrer en fonction de la mesure du taux de constituant effectuée, cette information étant prise en compte par le microprocesseur (20) et les moyens de mémoire (21) du dispositif pulvérisateur.

19. Ensemble selon l'une quelconque des revendications 13 à 18, caractérisé en ce que le médicament est de l'insuline.
